# EUROPEAN PATENT APPLICATION

(11) **EP 4 389 216 A2**
(43) Date of publication of application: **26.06.2024**
(21) Application number: 23193070.2
(22) Date of filing: 19.06.2018
(51) Int. Cl.: A61P 27/00

(54) **PEPTIDE COMPOSITIONS AND THERAPEUTIC USES**

(30) Priority: 19.06.2017 US 201762521984 P
(62) Divisional of application: 18820070.3
(71) Applicant: Allegro Pharmaceuticals, LLC, San Juan Capistrano, CA 92675 (US)
(72) Inventor: PARK, John, Y., Santa Ana, CA 92705 (US); KARAGEOZIAN, Hampar, L., San Juan Capistrano, CA 92675 (US); KARAGEOZIAN, Vicken, H., San Juan Capistrano, CA 92675 (US)
(74) Representative: CSY Herts

(57) **Abstract**

Peptide compositions and methods for inhibiting neovascularization or development of pathological or aberrant blood vessels in human or other animal subjects.

## Description

### Related Application

This European patent application is a divisional from European application no. 18820070.3 filed June 19, 2018, the entire disclosure of which is incorporated herein by reference. This patent application is based on a division of United States Patent Application Serial No. 16/012,706 filed June 19, 2018, which claims priority to United States Provisional Patent Application No. 62/521,984 entitled Peptide Compositions and Related Methods filed June 19, 2017, the entire disclosure of each such application being expressly incorporated herein by reference.

### Sequence Listing

**[0001.1]** The instant application contains a Sequence Listing which has been submitted electronically.

### Field of the Invention

The present invention relates generally to the fields of Biology and medicine and more particularly to peptide compositions and their methods of use.

### Background

Pursuant to 37 CFR 1.71(e), this patent document contains material which is subject to copyright protection and the owner of this patent document reserves all copyright rights whatsoever.

Throughout this patent application amino acids may be referred to interchangeably using the following names, three letter codes and single letter codes:

| **Amino Acid** | **Three letter code** | **Single Letter Code** |
|---|---|---|
| Alanine | Ala | A |
| Arginine | Arg | R |
| Asparagine | Asn | N |
| Aspartic Acid | Asp | D |
| Cysteine | Cys | C |
| Cysteic Acid | Cys(Acid) | - |
| Glutamic | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tyrosine | Tyr | Y |
| Valine | Val | V |

Applicant is developing the synthetic oligopeptide Glycinyl-Arginyl-Glycinyl-Cysteic(Acid)-Threonyl-Proline (SEQ ID NO: 1) (ALG-1001 or Luminate^{®}, Allegro Ophthalmics, LLC) which has been shown to inhibit a number of integrins and to have significant antiangiogenic, anti-inflammatory, neuroprotective and other effects. When administered to the eye, ALG-1001 can cause vitreolysis, posterior vitreo-retinal detachment (PVD) and is useable for treatment of eye disorders such as wet macular degeneration (WMD), dry macular degeneration (DMD), diabetic retinopathy (PDR), diabetic macular edema (DME) and vitreomacular traction (VMT). Further information regarding ALG-1001 and related compounds is found in United States Patent Nos. 9,018,352 entitled Peptide Compositions and Therapeutic Uses Thereof, 9,872,886 entitled *Compositions and Methods for Inhibiting Cellular Adhesion or Directing Diagnostic or Therapeutic Agents to RGD Binding Sites* and 9,896,480 entitled Integrin Receptor Antagonists and Their Methods of Use as well as pending United States Patent Application Serial No. 15/874,814 entitled *Therapeutic and Neuroprotective Peptides,* the entire disclosure of each such patent and patent application being expressly incorporated herein by reference.

Applicant has conceived of and synthesized the peptides listed in Table 1 below:

**TABLE 1**

| |
|---|
| GRGDTP (SEO ID NO: 2) |
| Cyclo-[RGD-DPhe-NMe-V] |
| GKGDTP (SEQ ID NO: 3) |
| Cyclo-[RGCys(acid)-F-NMe-V] (SEQ ID NO: 4) |
| GRADTP (SEQ ID NO: 5) |
| GRGCys(acid)TP. TCA salt (SEQ ID NO: 6) |
| GRGCys(acid)TP. Acetate (SEQ ID NO: 7) |
| GRGCys(acid)TP. HCL (SEQ ID NO: 8) |
| GRCys(acid)GTP (SEQ ID NO: 9) |
| GHGCys(acid)TP (SEQ ID NO: 10) |
| GRACys(acid)TP (SEQ ID NO: 11) |
| GKGCys(acid)TP (SEQ ID NO: 12) |
| GRGNTP (SEQ ID NO: 13) |
| GRGCTP (SEQ ID NO: 14) |
| GRGESP (SEQ ID NO: 15) |
| RGCys(acid)GGGDG (SEQ ID NO: 16) |
| GCys(acid)GRTP (SEQ ID NO: 17) |
| GDGRTP (SEQ ID NO: 18) |
| GRDGTP (SEQ ID NO: 19) |
| GRAETP (SEQ ID NO: 20) |
| GGCys(acid)RTP (SEQ ID NO: 21) |
| GCys(acid)ARTP (SEQ ID NO: 22) |
| RCys(acid)RGTP (SEQ ID NO: 23) |

As described below, Applicant has synthesized and performed initial testing on a number of additional novel peptides, a number of which exhibit therapeutic effects in *in vivo* tests.

### Summary

In accordance with the present invention, there are provided peptide compounds and methods for inhibiting neovascularization of the development of pathological or aberrant blood vessels in human or animal subjects.

In accordance with one aspect of the present invention, there are provided compositions of matter which comprise peptides that consist of or include an amino acid sequence having the formula:

**Y-X-Z**

wherein:
- Y =: R, H, K, Cys(acid), G or D;
- X =: G, A, Cys(acid), R, G, D or E; and
- Z =: Cys(acid), G, C, R, D, N or E.
Such peptides may comprise or consist of the amino acid sequences; R-G-Cys(Acid), R-R-Cys, R-CysAcid)-G, Cys(Acid)-R-G, Cys(Acid)-G-R, R-G-D, R-G-Cys(Acid). H-G-Cys(Acid), R-G-N, D-G-R, R-D-G, R-A-E, K-G-D, R-G-Cys(Acid)-G-G-G-D-G (SEQ ID NO: 16), Cyclo-{R-G-Cys(acid)-F-N-Me-V} (SEQ ID NO: 4), R-A-Cys (Acid), R-G-C, K-G-D, Cys(acid)-R-G, Cys(Acid)-G-R, Cyclo-{R-G-D-D-F-NMe-V} (SEQ ID NO: 24), H - G -Cys(acid) and salts thereof. Possible salts include but are not limited to acetate, trifluoroacetate (TFA) and hydrochloride salts. Such peptides are useful at least for inhibiting neovascularization of the development of pathological or aberrant blood vessels in human or animal subjects.

Further, in some embodiments, compositions of matter comprise or consist of peptides having General Formula 1 below:

**Gly-X-Thr-Pro**

wherein X is selected from: Arg-Ala-Cys(Acid); Arg-Gly-Cys; Arg-Asp-
Gly; Arg-Ala-Glu; Arg-Gly-Asn; Asp-Gly-Arg; Cys(acid)-Gly-Arg and Lys-Gly-Asp.

Further in accordance with the present invention, peptides of the present invention, or the synthetic oligopeptide Glycinyl-Arginyl-Glycinyl-Cysteic(Acid)-Threonyl-Proline (SEQ ID NO: 1), may be combined with Taurine and administered to a human or animal subject for the purpose of inhibiting neovascularization of the development of pathological or aberrant blood vessels
Still further in accordance with the present invention, there are provided methods for inhibiting neovascularization or the development of pathological or aberrant blood vessels in a human or animal subjects who are in need thereof, such methods comprising the step of administering to the subject a therapeutically effective amount of a composition comprising a peptide as summarized above. In some instances, such methods may be carried out to treat a disease or disorder of the eye wherein neovascularization or development of pathological or aberrant blood vessels occurs. Such diseases or disorders of the eye include but are not necessarily limited to: diabetic retinopathy, neovascular age-related macular degeneration, retinopathy of prematurity (ROP), sickle cell retinopathy, retinal vein occlusion, ischemia-induced retinopathy and certain inflammatory diseases of the eye..

Still further in accordance with the present invention, there are provided methods for inhibiting neovascularization or the development of pathological or aberrant blood vessels in human or animal subjects at locations outside of the eye. In some instances, such methods may be carried out to inhibit the growth or metastasis of a vascularized tumor.

Still further aspects and details of the present invention will be understood upon reading of the detailed description and examples set forth herebelow.

### Brief Description of the Drawings

Figure 1 is a bar graph of retinal neovascular area in ROP mouse eyes following treatment with either Gly-His-Gly-Cys(acid)-Thr-Pro (SEQ ID NO: 10) (Test Compound No. 14) or Control Peptide (Gly-Arg - Gly - Glu-Thr-Pro (SEQ ID NO: 26)).
Figure 2 is a bar graph of retinal neovascular area in ROP mouse eyes following treatment with either Gly-Arg-Gly-Cys(acid) -Thr-Pro TFA (SEQ ID NO: 27) (Positive Control), Gly-Arg-Ala-Cys-Thr-Pro (SEQ ID NO: 28) (Test Compound No. 3) or Control Peptide (Gly-Arg - Gly - Glu-Thr-Pro (SEQ ID NO: 26)).
Figure 3 is a bar graph of retinal neovascular area in ROP mouse eyes following treatment with either Gly-Arg-Gly-Cys(acid) -Thr-Pro TFA (SEQ ID NO: 27) (Test Compound No. 1/positive control), Gly-Arg-Ala-Asp-Thr-Pro (SEQ ID NO: 5) (Test Compound No. 23) or Control Peptide (Gly-Arg - Gly - Glu -Thr-Pro (SEQ ID NO: 26)).
Figure 4 is a bar graph of retinal neovascular area in ROP mouse eyes following treatment with either Gly-Arg-Gly-Cys(acid)-Thr-Pro TFA (SEQ ID NO: 27) (Positive Control), Gly-Arg-Ala-Cys(Acid)-Thr-Pro (SEQ ID NO: 11) (Test Compound No. 3) or Control Peptide (Gly-Arg - Gly - Glu-Thr-Pro (SEQ ID NO: 26)).
Figure 5 is a bar graph of retinal neovascular area in ROP mouse eyes following treatment with either Gly-Arg-Gly-Cys(acid)-Thr-Pro TFA (SEQ ID NO: 27) (Positive Control), Gly-Arg-Gly-Cys-Thr-Pro (SEQ ID NO: 14) (Test Compound No. 4) or Control Peptide (Gly-Arg - Gly - Glu-Thr-Pro (SEQ ID NO: 26)).
Figure 6 is a bar graph of retinal neovascular area in ROP mouse eyes following treatment with either Gly-Arg-Gly-Cys(acid)-Thr-Pro TFA (SEQ ID NO: 27) (Positive Control), Gly-Arg-Gly-Cys(acid)-Thr-Pro TFA (SEQ ID NO: 27) (Masked) (Test Compound No. 1) or Control Peptide (Gly-Arg - Gly - Glu-Thr-Pro (SEQ ID NO: 26)).
Figure 7 is a bar graph of retinal neovascular area in ROP mouse eyes following treatment with either Gly-Lys-Gly-Asp-Thr-Pro (SEQ ID NO: 3) (Test Compound No. 20) or Control Peptide (Gly-Arg - Gly - Glu-Thr-Pro (SEQ ID NO: 26)).
Figure 8 is a bar graph of retinal neovascular area in ROP mouse eyes following treatment with either Gly-His-Gly-Cys(Acid)-Thr-Pro (SEQ ID NO: 10) (Test Compound No. 14) or Control Peptide (Gly-Arg - Gly - Glu-Thr-Pro (SEQ ID NO: 26)).
Figure 9 is a bar graph of retinal neovascular area in ROP mouse eyes following treatment with either Gly-Lys-Gly-Cys(acid)-Thr-Pro (SEQ ID NO: 12) (Test Compound No. 6) or Control Peptide (Gly-Arg - Gly - Glu-Thr-Pro (SEQ ID NO: 26)).
Figure 10 is a bar graph of retinal neovascular area in ROP mouse eyes following treatment with either Gly-Arg-Gly-Cys(acid)-Thr-Pro TFA (SEQ ID NO: 27) (Positive Control), Gly-Arg-Cys(Acid)-Gly-Thr-Pro (SEQ ID NO: 9) (Test Compound No. 5) or Control Peptide (Gly-Arg - Gly - Glu-Thr-Pro (SEQ ID NO: 26)).
Figure 11 is a bar graph of retinal neovascular area in CNV mouse eyes following treatment with either Gly-Arg-Gly-Cys(acid)-Thr-Pro TFA (SEQ ID NO: 27) (Positive Control), Gly-Arg-Gly-Cys(Acid)-Thr-Pro Acetate (SEQ ID NO: 7) (Test Compound No. 2) or Control Peptide (Gly-Arg - Gly - Glu-Thr-Pro (SEQ ID NO: 26)).
Figure 12 is a bar graph of retinal neovascular area in ROP mouse eyes following treatment with either Gly-Arg-Gly-Cys(acid)-Thr-Pro TFA (SEQ ID NO: 27) (Positive Control), Gly-Arg-Gly-Cys(Acid)-Thr-Pro Acetate (SEQ ID NO: 7) (Test Compound No. 2) or Control Peptide (Gly-Arg - Gly - Glu-Thr-Pro (SEQ ID NO: 26)).
Figure 13 is a bar graph of retinal neovascular area in ROP mouse eyes following treatment with either Gly-Asp-Gly-Arg-Thr-Pro (SEQ ID NO: 18) (Test Compound No. 17) or Control Peptide (Gly-Arg - Gly - Glu-Thr-Pro (SEQ ID NO: 26)).
Figure 14 is a bar graph of retinal neovascular area in ROP mouse eyes following treatment with either Gly-Arg-Gly-Asp-Thr-Pro (SEQ ID NO: 2) (Test Compound No. 15) or Control Peptide (Gly-Arg - Gly - Glu-Thr-Pro (SEQ ID NO: 26)).
Figure 15 is a bar graph of retinal neovascular area in ROP mouse eyes following treatment with either Gly-Arg-Cys(Acid)-Gly-Thr-Pro (SEQ ID NO: 9) (Test Compound No. 18) or Control Peptide (Gly-Arg-Gly-Glu-Thr-Pro (SEQ ID NO: 26)).
Figure 16 is a bar graph of retinal neovascular area in ROP mouse eyes following treatment with either Arg-Gly-Cys(acid)-Gly-Gly-Asp-Gly (SEQ ID NO: 29) (Test Compound No. 7) or Control Peptide (Gly-Arg - Gly - Glu-Thr-Pro (SEQ ID NO: 26)). Figure discloses SEQ ID NO: 16.
Figure 17 is a bar graph of retinal neovascular area in ROP mouse eyes following treatment with either Gly-Arg-Ala-Glu-Thr-Pro (SEQ ID NO: 20) (Test Compound No. 19) or Control Peptide (Gly-Arg - Gly - Glu-Thr-Pro (SEQ ID NO: 26)).
Figure 18 is a bar graph of retinal neovascular area in ROP mouse eyes following treatment with either Gly-Gly-Cys(acid)-Arg-Thr-Pro (SEQ ID NO: 21) (Test Compound No. 11) or Control Peptide (Gly-Arg - Gly - Glu-Thr-Pro (SEQ ID NO: 26)).
Figure 19 is a bar graph of retinal neovascular area in ROP mouse eyes following treatment with either Gly-Cys(Acid)-Ala-Arg-Thr-Pro (SEQ ID NO: 22) (Test Compound No. 10) or Control Peptide (Gly-Arg - Gly - Glu-Thr-Pro (SEQ ID NO: 26)).
Figure 20 is a bar graph of retinal neovascular area in ROP mouse eyes following treatment with either Gly-Arg-Glu-Gly-Thr-Pro (SEQ ID NO: 30) (Test Compound No. 22) or Control Peptide (Gly-Arg - Gly - Glu-Thr-Pro (SEQ ID NO: 26)).
Figure 21 is a bar graph of retinal neovascular area in ROP mouse eyes following treatment with either Gly-Cys(acid)-Arg-Gly-Thr-Pro (SEQ ID NO: 31) (Test Compound No. 8) or Control Peptide (Gly-Arg - Gly - Glu-Thr-Pro (SEQ ID NO: 26)).
Figure 22 is a bar graph of retinal neovascular area in ROP mouse eyes following treatment with either Gly-Arg-Gly-Asn-Thr-Pro (SEQ ID NO: 13) (Test Compound No. 16) or Control Peptide (Gly-Arg - Gly - Glu-Thr-Pro (SEQ ID NO: 26)).
Figure 23 is a bar graph of retinal neovascular area in ROP mouse eyes following treatment with either Gly-Arg-Gly-Cys(acid)-Thr-Pro TFA (SEQ ID NO: 27) (Positive Control), Cyclo-{R-G-D-D-F-NMe-V} (SEQ ID NO: 24) (Test Compound No. 13) or Control Peptide (Arg - Gly - Glu-Thr-Pro (SEQ ID NO: 32)).
Figure 24 is a bar graph of retinal neovascular area in ROP mouse eyes following treatment with either Gly-Arg-Gly-Cys(acid)-Thr-Pro TFA (SEQ ID NO: 27) (Positive Control), Cyclo-{R-G-Cys(acid)-F-N-Me-V} (SEQ ID NO: 4) (Test Compound No. 12) or Control Peptide (Gly-Arg - Gly - Glu-Thr-Pro (SEQ ID NO: 26)).
Figure 25 is a bar graph of retinal neovascular area in ROP mouse eyes following treatment with either Gly-Arg-Gly-Cys(acid)-Thr-Pro TFA (SEQ ID NO: 27) (Positive Control), Gly-Cys(Acid) - Gly -Arg (SEQ ID NO: 33) (Test Compound No. 9) or Control Peptide (Gly-Arg - Gly - Glu (SEQ ID NO: 34)).
Figure 26 is a bar graph of retinal neovascular area in ROP mouse eyes following treatment with either Gly-His-Gly-Cys(Acid) (SEQ ID NO: 35) (Test Compound No. 14) or Control Peptide (Gly-Arg - Gly - Glu-Thr-Pro (SEQ ID NO: 26)).
Figure 27 is a bar graph of retinal neovascular area in ROP mouse eyes following treatment with either Gly-Arg-Gly-Cys(acid)-Thr-Pro TFA (SEQ ID NO: 27) (Positive Control), Taurine (Test Compound No. 25), Gly-Arg-Gly-Cys(acid) -Thr-Pro.TFA + Taurine (SEQ ID NO: 36) (Test Compound No. 24) or Control Peptide (Gly-Arg - Gly - Glu-Thr-Pro (SEQ ID NO: 26)).

### Detailed Description

The following detailed description and the accompanying drawings to which it refers are intended to describe some, but not necessarily all, examples or embodiments of the invention. The described embodiments are to be considered in all respects only as illustrative and not restrictive. The contents of this detailed description and the accompanying drawings do not limit the scope of the invention in any way.

A number of diseases and disorders are known to cause neovascularization or development of pathological or aberrant blood vessels, including diabetic retinopathy, neovascular age-related macular degeneration, retinopathy of prematurity (ROP), sickle cell retinopathy, retinal vein occlusion, ischemia-induced retinopathy, certain inflammatory diseases of the eye and the growth or metastasis of a vascularized tumors. Applicant has discovered a number of compounds that are shown to be active in an anti-neovascularization mouse ROP model as described below. On this basis, such compounds are potentially useful in the treatment of diseases and disorders which are known to cause neovascularization or development of pathological or aberrant blood vessels, including but not limited to those diseases and disorders listed above.

Each test compound was prepared in sterile water for injection, containing 0.08mg/100µL of sodium chloride and 0.005mg/100µL of trisodium citrate, the concentration of the peptide was at a concentration of 2.0mg/100µL and pH = 2.7 and dispensed by sterile filtration into sterile vials. The Taurine test compound was obtained from Sigma Aldrich company, which was >99% pure, and prepared the same way as mentioned previously, having a concentration of 3.0mg/100µL. The R-G-Cys(acid) at 2.0mg/100µL + Taurine at 3.0mg/100µL were prepared the same way as mentioned above

To screen the test compounds for activity against ischemia-induced retinal neovascularization, the well-established model of retinopathy of prematurity (ROP) in mice was used. Litters of C57BI/6 mice were placed in 75% oxygen at postnatal day (P) 7, returned to room air at day (P) 12. The Pups were randomly assigned to treatment groups of 4 to 10 animals per group. The pups were treated as follows: Treatment eyes were treated by intravitreal injection of 1.0 microliters of solution containing 20 micrograms of Test Compound.

On post-natal day 17, 5 days after intravitreal injection, the animals were sacrificed, the retinas were flat mounted and the area of neovascularization in each retina was determined by Fluorescein-dextran image analysis.

Applicant has identified the amino acid sequence R-G-Cysteic(Acid) as an active motif of the oligopeptide Glycinyl-Arginyl-Glycinyl-Cysteic(Acid)-Threonyl-Proline (SEQ ID NO: 1) (ALG-1001 or Luminate^{®}, Allegro Ophthalmics, LLC) that provides certain pharmacologic activity. The trifluoroacetate (TFA) and acetate salts of the R-G-Cysteic(Acid) tripeptide (Test Compound Nos. 1 and 2 ) were tested in both the ROP Mouse Model as described above as well as in a mouse model of choroidal neovascularization induced by laser photocoagulation ("CNV Mouse Model"), as generally as described in Lambert, V., et al., Laser-Induced Choroidal Neovascarization Model to Study Age Related Macular Degeneration in Mice, Nature Protocols, 8; 2197-2211 (2013). Animals assigned to "Control" groups were treated by intravitreal injection of Gly-Arg-Gly-Glu-Thr-Pro (SEQ ID NO: 26) (Inactive Control Peptide), which is known to be inactive in this model. In some of the experiments, an additional "Positive Control" group was included. Animals assigned to a "Positive Control" group were treated by intravitreal injection of Gly-Arg-Gly-Cys(acid) -Thr-Pro · TFA (SEQ ID NO: 37), which is known to be active in this model.

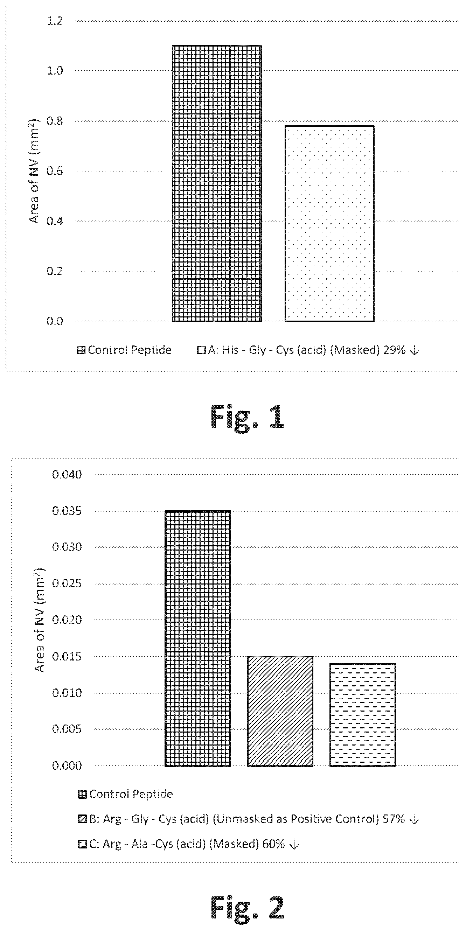

The following Table 2 summarizes the neovascularization inhibiting effect of each Test Compound at the dose tested. In each instance, the data was obtained using the ROP Mouse Model, except for the two table entries specifically labeled "CNV". Only those table entries labeled "CNV" show data obtained from the CNV Mouse Model. Bar graphs showing the test results summarized in Table 2 are also provided herewith as Figures 1 through 27. Where indicated in the figures, the tests were performed in a blinded manner such that the persons performing the testing did not know the identity or structure of each test compound.

**TABLE 2**

| ***SUPPRESSION OF RETINAL NEOVASCULARIZATION IN MOUSE MODEL OF ROP (ISCHEMIC) RETINOPATHY*** | | | |
|---|---|---|---|
| **Test Compound Number** | **Test Compound** | **Mean % Reduction of Retinal Neovascularization In ROP Model** | **Activity At Dose Tested** |
| 1 | G-R - G - Cys(acid)-T-P.TFA -ROP (SEQ ID NO: 38) | 61 | Active |
| 1(CNV) | G-R- G - Cys(acid) -T-P.TFA - CNV (SEQ ID NO: 39) | 49 - Fig 11 | Active |
| 2(CNV) | G-R- G - Cys(acid) -T-P.Acetate - CNV (SEQ ID NO: 40) | 56 - Fig 11 | Active |
| 2 | G-R- G - Cys(acid) -T-P.Acetate - ROP (SEQ ID NO: 41) | 72 | Active |
| 3 | G-R - A - Cys (acid) -T-P.TFA (SEQ ID NO: 42) | 60 | Active |
| 4 | G-R - G - Cysteine-T-P.TFA (SEQ ID NO: 43) | 66 | Active |
| 5 | G-R - Cys(acid) - G-T-P.TFA (SEQ ID NO: 44) | 33 | Slightly Active |
| 6 | G-K - G - Cys (acid) -T-P.TFA (SEQ ID NO: 45) | 0 | Not Active |
| 7 | R - G - Cys(acid)-G-G-G-D-G.TFA (SEQ ID NO: 46) | 62 | Active |
| 8 | G-Cys(acid) - R - G-T-P.TFA (SEQ ID NO: 47) | 21 | Slightly Active |
| 9 | G-Cys(acid) - G - R-T-P.TFA (SEQ ID NO: 48) | 63 | Active |
| 10 | G-Cys(acid) - A - R-T-P.TFA (SEQ ID NO: 49) | 0 | Not Active |
| 11 | G-G - Cys(acid) - R-T-P.TFA (SEQ ID NO: 50) | 0 | Not Active |
| 12 | Cyclo-{R-G-Cys(acid)-F-N-Me-V} Acetate (SEQ ID NO: 51) | 57 | Active |
| 13 | Cyclo-{R-G-D-D-F-NMe-V}.TFA (SEQ ID NO: 52) | 75 | Active |
| 14 | G-H - G -Cys(acid) -T-P.TFA (SEQ ID NO: 53) | 28 | Slightly Active |
| 15 | G-R - G - D-T-P.TFA (SEQ ID NO: 54) | 37 | Slightly Active |
| 16 | G-R - G - N-T-P.TFA (SEQ ID NO: 55) | 64 | Active |
| 17 | G-D - G - R-T-P.TFA (SEQ ID NO: 56) | 56 | Active |
| 18 | G-R - D - G-T-P.TFA (SEQ ID NO: 57) | 44 | Active |
| 19 | G-R - A - E-T-P.TFA (SEQ ID NO: 58) | 63 | Active |
| 20 | G-K - G - D-T-P.TFA (SEQ ID NO: 59) | 40 | Active |
| 21 | G-R - G - E-T-P.TFA (SEQ ID NO: 60) | 0 | Not Active |
| 22 | G-R - E - G-T-P.TFA (SEQ ID NO: 61) | 0 | Not Active |
| 23 | G-R - A - D-T-P.TFA (SEQ ID NO: 25) | 0 | Not Active |
| 24 | G-R-G-Cys(acid) -T-P.TFA +Taurine (SEQ ID NO: 36) | 58 | Active |
| 25 | Taurine | 33 | Slightly Active |

In each of the Test Compounds, except for cyclic Test Compounds No. 12 and 13, the RGCys(acid) active motif of the positive control GRGCys(acid)TP (SEQ ID NO: 1) (ALG-1001) was rearranged and/or replaced by a different three amino acid motif having an amino acid sequence defined in General Formula 2 (below). This resulted in pentapeptide test compounds having general formulas defined by General Formula 1 (above).

***General Formula** 2* **Y-X-Z**

**Wherein:**
- **Y =**: **R*, H, K, Cys(acid), G or D;**
- **X =**: **G*, A, Cys(acid), R, G, D or E; and**
- **Z =**: **Cys*, G, Cysteine, R, D, N or E.**

**indicates component of the RGCys(acid) binding motif of tripeptide in GRGCys(acid)TP (SEQ ID NO: 1) (ALG-1001), which was used as a Positive Control.**

Based on the results of the ROP and CNV testing summarized above, the the presence of Arginine, Alanine and Cysteic Acid in the GRGCys(acid)TP peptide (SEQ ID NO: 1) (ALG-1001/Luminate) plays an important role in the suppression of the neovascularization, notably the sequence of R-G-Cys and R-A-Cys. Furthermore, in the presence of arginine, replacement of Cysteic (Acid) by a neutral amino acid exhibited a strong suppressive effect in these experiments.

Based on the initial data set forth herein, certain structure/activity relationships are suggested in relation to specific changes made to the R-G-Cysteic Acid active motif. For example, when the amino acid R (i.e., the Y Component) of the R-G-Cysteic(Acid) binding motif is replaced by a basic amino acid or acidic amino acid, the peptide's anti-neovascularization effects diminish, whereas in the presence of arginine in the active motif aspartic acid as Component Y appears to promote the peptide's anti-neovascularization effects.

When amino acid G (i.e., the X Component) of the R-G-Cysteic Acid active motif is replaced by a basic or acidic amino acid, the peptide's anti-neovascularization effects decrease. However, in the presence of arginine (a strong hydrogen bonding), two carbon length-space for hydrophobic interaction (Alanine and Aspartic Acid) may not influence the peptide's anti-neovascularization effects.

When Cys(Acid) (i.e., the Z Component) of the R-G-Cysteic(Acid) active motif is replaced by a neutral amino acid, the peptide's neovascularization inhibiting activity increases whereas replacement of the Z component by acidic or basic amino acids causes the neovascularization inhibiting activity to decrease.

All indications are that the R-G-Cysteic(Acid) of the oligopeptide Glycinyl-Arginyl-Glycinyl-Cysteic(Acid)-Threonyl-Proline (SEQ ID NO: 1) (ALG-1001 or Luminate^{®}, Allegro Ophthalmics, LLC) is important for suppression of neovascularization. Also, addition of three parts taurine to one part of the Glycinyl-Arginyl-Glycinyl-Cysteic(Acid)-Threonyl-Proline (SEQ ID NO: 1) (ALG-1001) improves the neovascularization suppressing activity.

It is to be appreciated that, although the invention has been described hereabove with reference to certain examples or embodiments of the invention, various additions, deletions, alterations and modifications may be made to those described examples and embodiments without departing from the intended spirit and scope of the invention. For example, any elements, steps, members, components, compositions, reactants, parts or portions of one embodiment or example may be incorporated into or used with another embodiment or example, unless otherwise specified or unless doing so would render that embodiment or example unsuitable for its intended use. Also, where the steps of a method or process have been described or listed in a particular order, the order of such steps may be changed unless otherwise specified or unless doing so would render the method or process unsuitable for its intended purpose. Additionally, the elements, steps, members, components, compositions, reactants, parts or portions of any invention or example described herein may optionally exist or be utilized in the absence or substantial absence of any other element, step, member, component, composition, reactant, part or portion unless otherwise noted. All reasonable additions, deletions, modifications and alterations are to be considered equivalents of the described examples and embodiments and are to be included within the scope of the following claims.

### Annex A

The following, on pages 1A to 12A, is a copy of the description of the parent application of the present divisional as filed (European application no. 18820070.3 filed June 19, 2018), which is annexed herewith for the purposes of completeness, should it be needed during prosecution, including for basis/support purposes. The Applicant reserves the right to introduce any subject-matter from the Annexes to the rest of the specification.

### PEPTIDE COMPOSITIONS AND RELATED METHODS

### Related Application

This patent application claims priority to United States Provisional Patent Application No. 62/521,984 entitled Peptide Compositions and Related Methods filed June 19, 2017, the entire disclosure of which is expressly incorporated herein by reference.

### Field of the Invention

The present invention relates generally to the fields of Biology and medicine and more particularly to peptide compositions and their methods of use.

### Background

Pursuant to 37 CFR 1.71(e), this patent document contains material which is subject to copyright protection and the owner of this patent document reserves all copyright rights whatsoever.

Throughout this patent application amino acids may be referred to interchangeably using the following names, three letter codes and single letter codes:

| **Amino Acid** | **Three letter code** | **Single Letter Code** |
|---|---|---|
| Alanine | Ala | A |
| Arginine | Arg | R |
| Asparagine | Asn | N |
| Aspartic Acid | Asp | D |
| Cysteine | Cys | c |
| Cysteic Acid | Cys(Acid) | - |
| Glutamic | Glu | E |
| Glutamine | Gin | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tyrosine | Tyr | Y |
| Valine | Val | ν |

Applicant is developing the synthetic oligopeptide Glycinyl-Arginyl-Glycinyl-Cysteic(Acid)-Threonyl-Proline (ALG-1001 or Laminate^{®}, Allegro Ophthalmics, LLC) which has been shown to inhibit a number of integrins and to have significant antiangiogenic, anti-inflammatory, neuroprotective and other effects. When administered to the eye, ALG-1001 can cause vitreolysis, posterior vitreo-retinal detachment (PVD) and is useable for treatment of eye disorders such as wet macular degeneration (WMD), dry macular degeneration (DMD), diabetic retinopathy (PDR), diabetic macular edema (DME) and vitreomacular traction (VMT). Further information regarding ALG-1001 and related compounds is found in United States Patent Nos. 9,018,352 entitled Peptide Compositions and Therapeutic Uses Thereof, 9,872,886 entitled *Compositions and Methods for Inhibiting Cellular Adhesion or Directing Diagnostic or Therapeutic Agents to RGD Binding Sites* and 9,896,480 entitled Integrin Receptor Antagonists and Their Methods of Use as well as pending United States Patent Application Serial No. 15/874,814 entitled *Therapeutic and Neuroprotective Peptides,* the entire disclosure of each such patent and patent application being expressly incorporated herein by reference.

As described below, Applicant has synthesized and performed initial testing on a number of additional novel peptides, a number of which exhibit therapeutic effects in *in vivo* tests.

### Summary

In accordance with the present invention, there are provided peptide compounds and methods for inhibiting neovascularization of the development of pathological or aberrant blood vessels in human or animal subjects.

In accordance with one aspect of the present invention, there are provided compositions of matter which comprise peptides that consist of or include an amino acid sequence having the formula:

**Y-X-Z**

wherein:
- Y =: R, H, K, Cys(acid), G or D;
- X =: G, A, Cys(acid), R, G, D or E; and
- Z =: Cys(acid), G, C, R, D, N or E.
Such peptides may comprise or consist of the amino acid sequences; R-G-Cys(Acid), R-R-Cys, R-CysAcid)-G, Cys(Acid)-R-G, Cys(Acid)-G-R, R-G-D, R-G-Cys(Acid). H-G-Cys(Acid), R-G-N, D-G-R, R-D-G, R-A-E, K-G-D, R-G.... Cys(Acid)-G-G-G-D-G, Cyclo-{R-G-Cys(acid)-F-N-Me-V}, R-A-Cys (Acid), R-G-C, K-G-D, Cys(acid)-R-G, Cys(Acid)-G-R, Cyclo-{R-G-D-D-F-NMe-V}, H - G -Cys(acid) and salts thereof. Possible salts include but are not limited to acetate, trifluoroacetate (TFA) and hydrochloride salts. Such peptides are useful at least for inhibiting neovascularization of the development of pathological or aberrant blood vessels in human or animal subjects
Further in accordance with the present invention, peptides of the present invention, or the synthetic oligopeptide Glycinyl-Arginyl-Glycinyl-Cysteic(Acid)-Threonyl-Proline, may be combined with Taurine and administered to a human or animal subject for the purpose of inhibiting neovascularization of the development of pathological or aberrant blood vessels

Still further in accordance with the present invention, there are provided methods for inhibiting neovascularization or the development of pathological or aberrant blood vessels in a human or animal subjects who are in need thereof, such methods comprising the step of administering to the subject a therapeutically effective amount of a composition comprising a peptide as summarized above. In some instances, such methods may be carried out to treat a disease or disorder of the eye wherein neovascularization or development of pathological or aberrant blood vessels occurs. Such diseases or disorders of the eye include but are not necessarily limited to: diabetic retinopathy, neovascular age-related macular degeneration, retinopathy of prematurity (ROP), sickle cell retinopathy, retinal vein occlusion, ischemia-induced retinopathy and certain inflammatory diseases of the eye..

Still further in accordance with the present invention, there are provided methods for inhibiting neovascularization or the development of pathological or aberrant blood vessels in human or animal subjects at locations outside of the eye. In some instances, such methods may be carried out to inhibit the growth or metastasis of a vascularized tumor.

Still further aspects and details of the present invention will be understood upon reading of the detailed description and examples set forth herebelow.

### Brief Description of the Drawings

Figure 1 is a bar graph of retinal neovascular area in ROP mouse eyes following treatment with either His-Gly-Cys(acid) (Test Compound No. 14) or Control Peptide (Arg - Gly - Glu).
Figure 2 is a bar graph of retinal neovascular area in ROP mouse eyes following treatment with either Arg-Gly-Cys(acid)TFA (Positive Control), Arg-Ala-Cys (Test Compound No. 3) or Control Peptide (Arg - Gly - Glu).

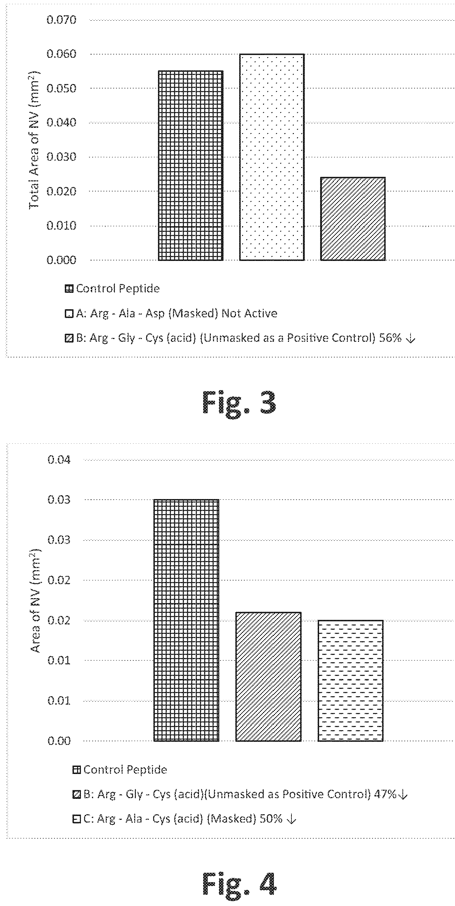

Figure 3 is a bar graph of retinal neovascular area in ROP mouse eyes following treatment with either Arg-Gly-Cys(acid)TFA (Test Compound No. 1/positive control), Arg-Ala-Asp (Test Compound No. 23) or Control Peptide (Arg Gly - Glu).
Figure 4 is a bar graph of retinal neovascular area in ROP mouse eyes following treatment with either Arg-Gly-Cys(acid)TFA (Positive Control), Arg-Ala-Cys(Acid) (Test Compound No. 3) or Control Peptide (Arg - Gly - Glu).

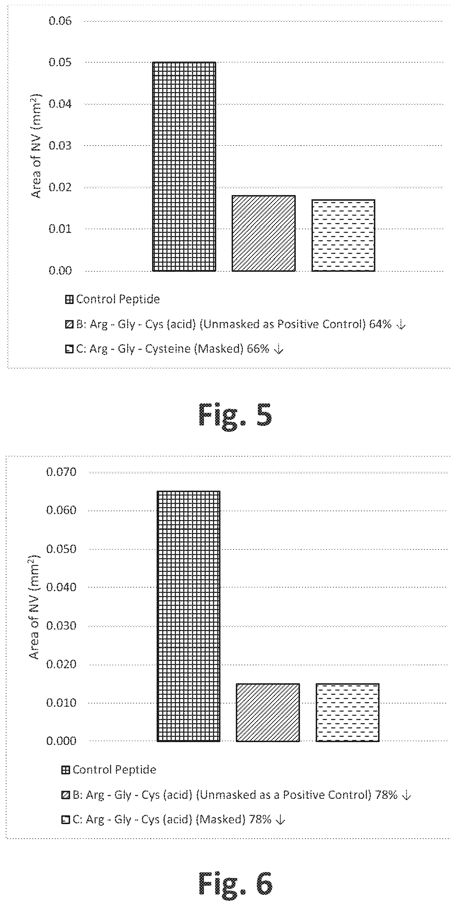

Figure 5 is a bar graph of retinal neovascular area in ROP mouse eyes following treatment with either Arg-Gly-Cys(acid)TFA (Positive Control), Arg-Gly-Cys (Test Compound No. 4) or Control Peptide (Arg - Gly - Glu).
Figure 6 is a bar graph of retinal neovascular area in ROP mouse eyes following treatment with either Arg-Gly-Cys(acid)TFA (Positive Control), Arg-Gly-Cys(acid)TFA (Masked) (Test Compound No. 1) or Control Peptide (Arg - Gly - Glu).

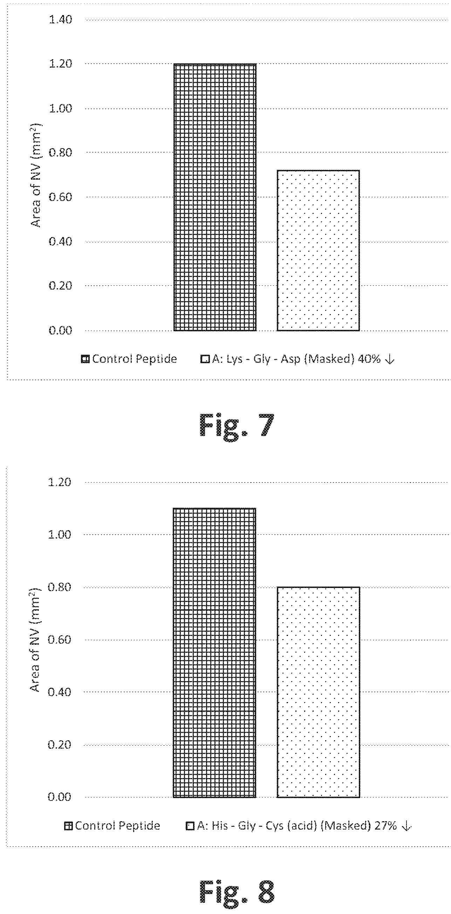

Figure 7 is a bar graph of retinal neovascular area in ROP mouse eyes following treatment with either Lys-Glys-Asp (Test Compound No. 20) or Control Peptide (Arg - Gly - Glu).
Figure 8 is a bar graph of retinal neovascular area in ROP mouse eyes following treatment with either His-Gly-Cys(Acid) (Test Compound No. 14) or Control Peptide (Arg - Gly - Glu).

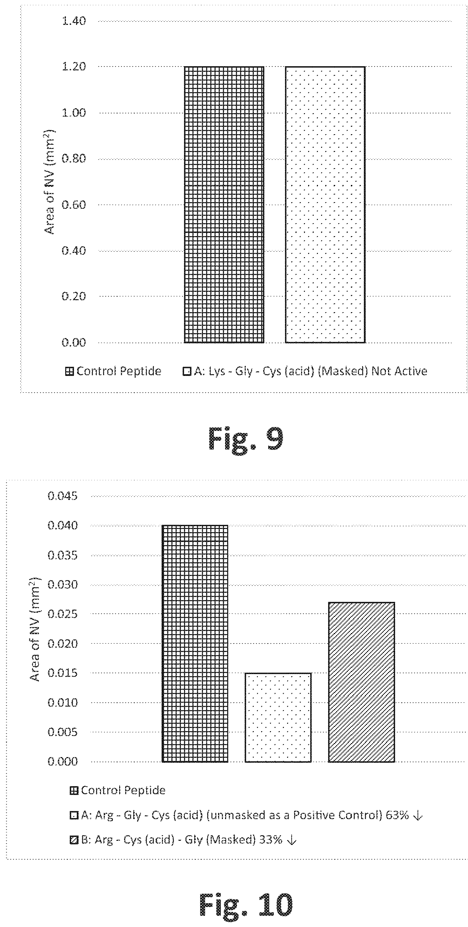

Figure 9 is a bar graph of retinal neovascular area in ROP mouse eyes following treatment with either Lys-Gly-Cys(acid) (Test Compound No. 6) or Control Peptide (Arg - Gly - Glu).
Figure 10 is a bar graph of retinal neovascular area in ROP mouse eyes following treatment with either Arg-Gly-Cys(acid)TFA (Positive Control), Arg-Cys(Acid)-Gly (Test Compound No. 5) or Control Peptide (Arg - Gly - Glu).

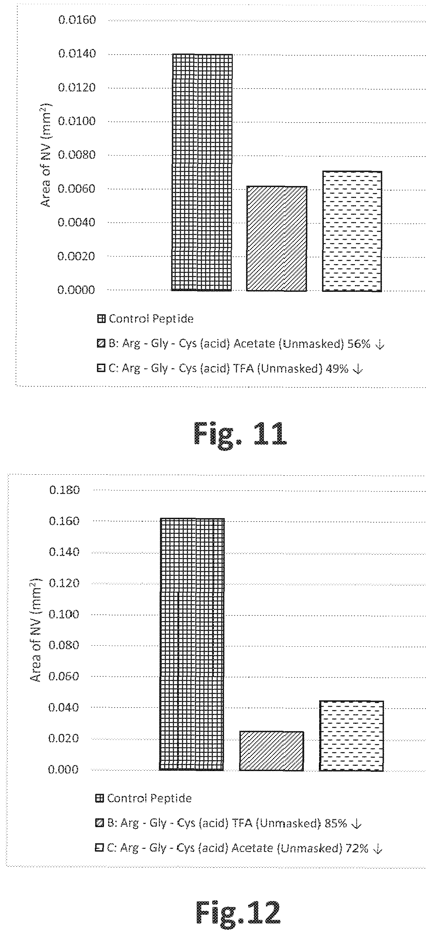

Figure 11 is a bar graph of retinal neovascular area in CNV mouse eyes following treatment with either Arg-Gly-Cys(acid)TFA (Positive Control), Arg-Gly-Cys(Acid) Acetate (Test Compound No. 2) or Control Peptide (Arg - Gly - Glu).
Figure 12 is a bar graph of retinal neovascular area in ROP mouse eyes following treatment with either Arg-Gly-Cys(acid)TFA (Positive Control), Arg-Gly-Cys(Acid) Acetate (Test Compound No. 2) or Control Peptide (Arg - Gly - Glu).

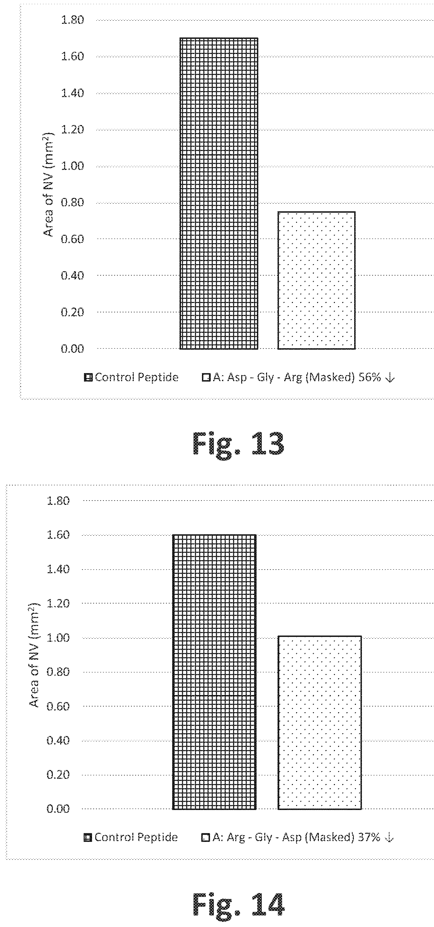

Figure 13 is a bar graph of retinal neovascular area in ROP mouse eyes following treatment with either Asp-Gly-Arg (Test Compound No. 17) or Control Peptide (Arg - Gly - Glu).
Figure 14 is a bar graph of retinal neovascular area in ROP mouse eyes following treatment with either Arg-Gly-Asp (Test Compound No. 15) or Control Peptide (Arg - Gly - Glu).

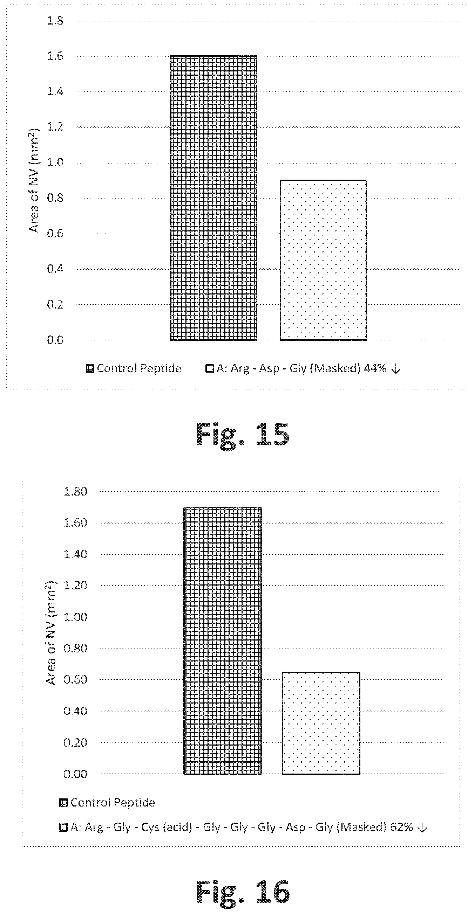

Figure 15 is a bar graph of retinal neovascular area in ROP mouse eyes following treatment with either Arg-Cys(Acid)-Gly (Test Compound No. 18) or Control Peptide (Arg - Gly - Glu).
Figure 16 is a bar graph of retinal neovascular area in ROP mouse eyes following treatment with either Arg-Gly-Cys(acid)-Gly-Gly-Asp-Gly (Test Compound No. 7) or Control Peptide (Arg - Gly - Glu).

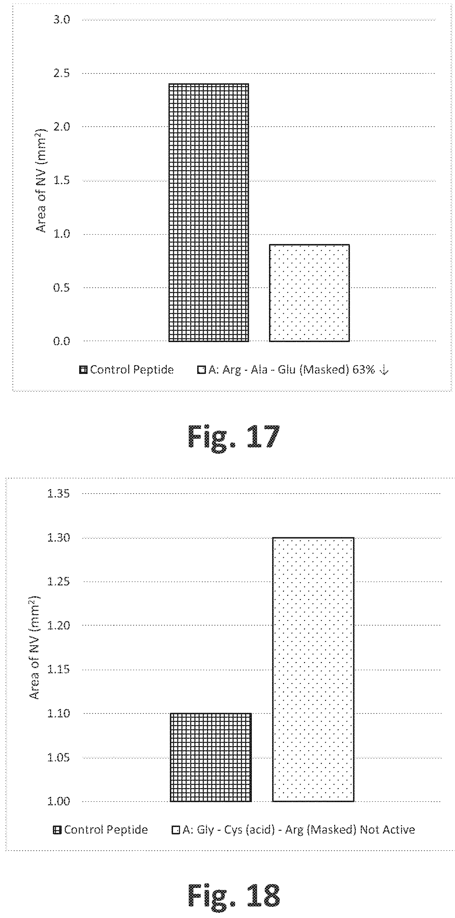

Figure 17 is a bar graph of retinal neovascular area in ROP mouse eyes following treatment with either Arg-Ala-Glu (Test Compound No. 19) or Control Peptide (Arg - Gly - Glu).
Figure 18 is a bar graph of retinal neovascular area in ROP mouse eyes following treatment with either Gly-Cys(acid)-Arg (Test Compound No. 11) or Control Peptide (Arg - Gly - Glu).

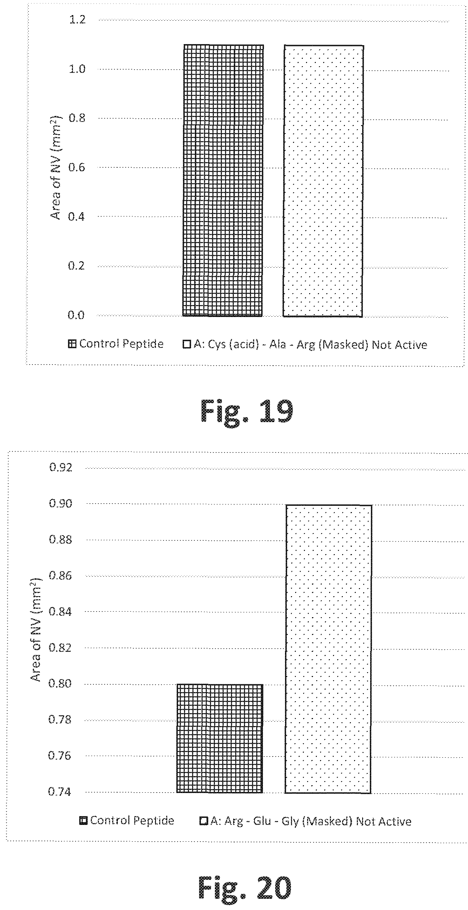

Figure 19 is a bar graph of retinal neovascular area in ROP mouse eyes following treatment with either Cys(Acid)-Ala-Arg (Test Compound No. 10) or Control Peptide (Arg - Gly - Glu).
Figure 20 is a bar graph of retinal neovascular area in ROP mouse eyes following treatment with either Arg-Glu-Gly (Test Compound No. 22) or Control Peptide (Arg - Gly - Glu).

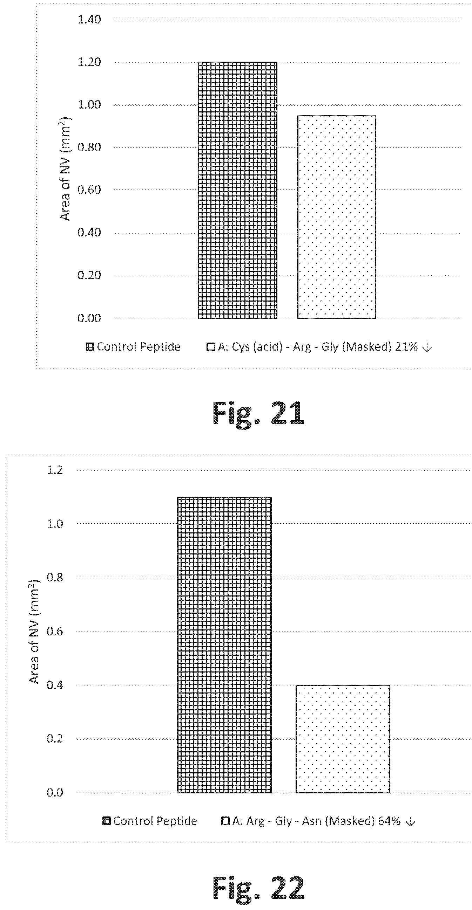

Figure 21 is a bar graph of retinal neovascular area in ROP mouse eyes following treatment with either Cys(acid)-Arg-Gly (Test Compound No. 8) or Control Peptide (Arg - Gly - Glu).
Figure 22 is a bar graph of retinal neovascular area in ROP mouse eyes following treatment with either Arg-Gly-Asn (Test Compound No. 16) or Control Peptide (Arg - Gly - Glu).

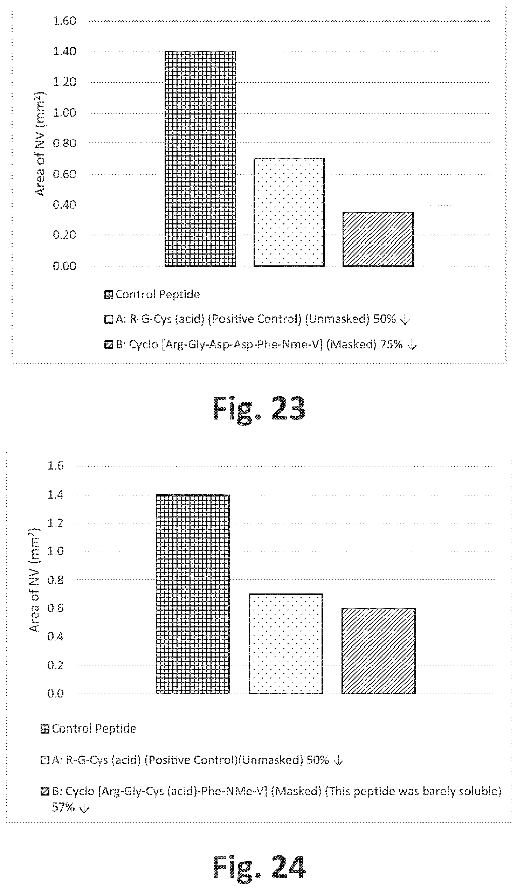

Figure 23 is a bar graph of retinal neovascular area in ROP mouse eyes following treatment with either Arg-Gly-Cys(acid)TFA (Positive Control), Cyclo-{R-G-D-D-F-NMe-V} (Test Compound No. 13) or Control Peptide (Arg - Gly - Glu).
Figure 24 is a bar graph of retinal neovascular area in ROP mouse eyes following treatment with either Arg-Gly-Cys(acid)TFA (Positive Control), Cyclo-{R-G-Cys(acid)-F-N-Me-V} (Test Compound No. 12) or Control Peptide (Arg - Gly - Glu).

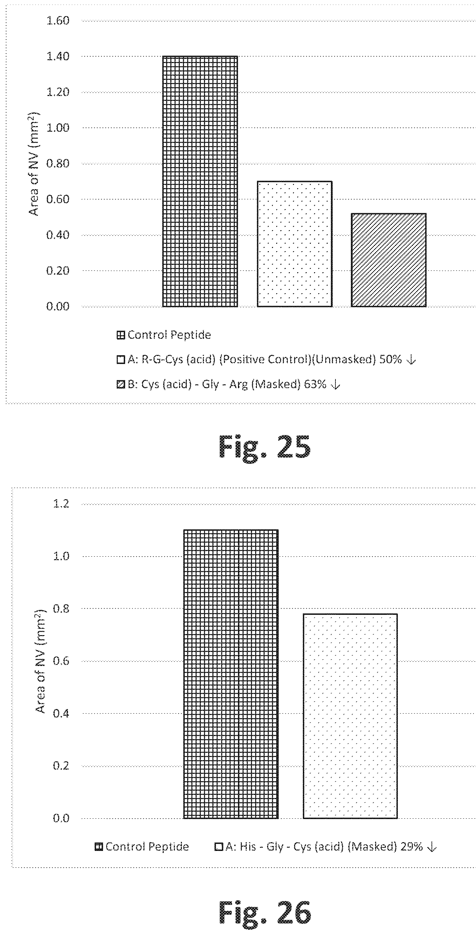

Figure 25 is a bar graph of retinal neovascular area in ROP mouse eyes following treatment with either Arg-Gly-Cys(acid)TFA (Positive Control), Cys(Acid) - Gly -Arg (Test Compound No. 9) or Control Peptide (Arg - Gly - Glu).
Figure 26 is a bar graph of retinal neovascular area in ROP mouse eyes following treatment with either His-Gly-Cys(Acid) (Test Compound No. 14) or Control Peptide (Arg - Gly - Glu).

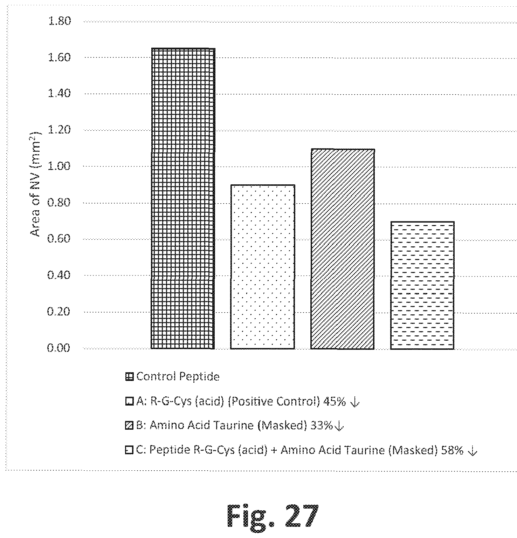

Figure 27 is a bar graph of retinal neovascular area in ROP mouse eyes following treatment with either Arg-Gly-Cys(acid)TFA (Positive Control), Taurine (Test Compound No. 25), Arg-Gly-Cys(acid).TFA + Taurine (Test Compound No. 24) or Control Peptide (Arg - Gly - Glu).

### Detailed Description

The following detailed description and the accompanying drawings to which it refers are intended to describe some, but not necessarily all, examples or embodiments of the invention. The described embodiments are to be considered in all respects only as illustrative and not restrictive. The contents of this detailed description and the accompanying drawings do not limit the scope of the invention in any way.

A number of diseases and disorders are known to cause neovascularization or development of pathological or aberrant blood vessels, including diabetic retinopathy, neovascular age-related macular degeneration, retinopathy of prematurity (ROP), sickle cell retinopathy, retinal vein occlusion, ischemia-induced retinopathy, certain inflammatory diseases of the eye and the growth or metastasis of a vascularized tumors. Applicant has discovered a number of compounds that are shown to be active in an anti-neovascularization mouse ROP model as described below. On this basis, such compounds are potentially useful in the treatment of diseases and disorders which are known to cause neovascularization or development of pathological or aberrant blood vessels, including but not limited to those diseases and disorders listed above.

Each test compound was prepared in sterile water for injection, containing 0.08mg/100pL of sodium chloride and 0.005mg/100µL of trisodium citrate, the concentration of the peptide was at a concentration of 2.0mg/100µL and pH = 2.7 and dispensed by sterile filtration into sterile vials. The Taurine test compound was obtained from Sigma Aldrich company, which was >99% pure, and prepared the same way as mentioned previously, having a concentration of 3.0mg/100pL. The R-G-Cys(acid) at 2.0mg/100µL + Taurine at 3.0mg/100µL were prepared the same way as mentioned above
To screen the test compounds for activity against ischemia-induced retinal neovascularization, the well-established model of retinopathy of prematurity (ROP) in mice was used. Litters of C57BI/6 mice were placed in 75% oxygen at postnatal day (P) 7, returned to room air at day (P) 12. The Pups were randomly assigned to treatment groups of 4 to 10 animals per group. The pups were treated as follows: Treatment eyes were treated by intravitreal injection of 1.0 microliters of solution containing 20 micrograms of Test Compound.

On post-natal day 17, 5 days after intravitreal injection, the animals were sacrificed, the retinas were flat mounted and the area of neovascularization in each retina was determined by Fluorescein-dextran image analysis.

Applicant has identified the tripeptide R-G-Cysteic(Acid) as an integrin binding motif of the oligopeptide Glycinyl-Arginyl-Glycinyl-Cysteic(Acid)-Threonyl-Proline (ALG-1001 or Luminate^{®}, Allegro Ophthalmics, LLC). The trifluoroacetate (TFA) and acetate salts of the R-G-Cysteic(Acid) tripeptide (Test Compound Nos. 1 and 2 ) were tested in both the ROP Mouse Model as described above as well as in a mouse model of choroidal neovascularization induced by laser photocoagulation ("CNV Mouse Model"), as generally as described in Lambert, V., et al., Laser Induced Choroidal Neovascarization Model to Study Age Related Macular Degeneration in Mice, Nature Protocols, 8; 2197-2211 (2013). Animals assigned to "Control" groups were treated by intravitreal injection of Arg - Gly - Glu (Control Peptide), which is known to be inactive. In some of the experiments, an additional "Positive Control" group was included. Animals assigned to a "Positive Control" group were treated by intravitreal injection of Arg-Gly-Cys(acid)TFA, which is known to be active.

The following Table 1 summarizes the neovascularization inhibiting effect of each Test Compound at the dose tested. In each instance, the data was obtained using the ROP Mouse Model, except for the two table entries specifically labeled "CNV". Only those table entries labeled "CNV" show data obtained from the CNV Mouse Model. Bar graphs showing the test results summarized in Table 1 are also provided herewith as Figures 1 through 27. Where indicated in the figures, the tests were performed in a blinded manner such that the persons performing the testing did not know the identity or structure of each test compound.

**TABLE 1**

| ***SUPPRESSION OF RETINAL NEOVASCULARIZATION IN MOUSE MODEL OF ROP (ISCHEMIC) RETINOPATHY*** | | | |
|---|---|---|---|
| **Test Compound Number** | **Test Compound** | **Mean % Reduction of Retinal Neovascularization in ROP Model** | **Activity At Dose Tested** |
| 1 | R - G - Cys(acid).TFA -ROP | 61 | Active |
| 1(CNV) | R- G - Cys(acid).TFA - CNV | 49 - Fig 11 | Active |
| 2(CNV) | R- G - Cys(acid).Acetate - CNV | 56 - Fig 11 | Active |
| 2 | R- G - Cys(acid).Acetate - ROP | 72 | Active |
| 3 | R - A - Cys (acid).TFA | 60 | Active |
| 4 | R - G - Cysteine.TFA | 66 | Active |
| 5 | R - Cys(acid) - G.TFA | 33 | Slightly Active |
| 6 | K - G - Cys (acid).TFA | 0 | Not Active |
| 7 | R - G - Cys(acid)-G-G-G-D-G.TFA | 62 | Active |
| 8 | Cys(acid) - R - G.TFA | 21 | Slightly Active |
| 9 | Cys(acid) - G - R.TFA | 63 | Active |
| 10 | Cys(acid) - A - R.TFA | 0 | Not Active |
| 11 | G - Cys(acid) - R.TFA | 0 | Not Active |
| 12 | Cyclo-{R-G-Cys(acid)-F-N-Me-V; Acetate | 57 | Active |
| 13 | Cyclo-{R-G-D-D-F-NMe-V}.TFA | 75 | Active |
| 14 | H - G -Cys(acid).TFA | 2.8 | Slightly Active |
| 15 | R - G - D.TFA | 37 | Slightly Active |
| 16 | R - G - N.TFA | 64 | Active |
| 17 | D - G - R.TFA | 56 | Active |
| 18 | R ... D - G.TFA | 44 | Active |
| 19 | R - A - E.TFA | 63 | Active |
| 20 | K - 6 - D.TFA | 40 | Active |
| 21 | R - G - E.TFA | 0 | Not Active |
| 22 | R - E - G.TFA | 0 | Not Active |
| 23 | R - A - D.TFA | 0 | Not Active |
| 24 | R-G-Cys(acid).TFA +Taurine | 58 | Active |
| 25 | Taurine | 33 | Slightly Active |

In some of the Test Compounds, the amino acid sequence of the binding motif RGCys(acid) tripeptide in GRGCys(acid)TP (ALG-1001) was rearranged and/or replaced by other basic, acidic and neutral amino acids. Based on the results of the ROP and CNV testing summarized above, the result indicates that the presence of Arginine, Alanine and Cysteic Acid in the GRGCys(acid)TP peptide (ALG-1001/Luminate) plays an important role in the suppression of the neovascularization, notably the sequence of R-G-Cys and R-A-Cys. Furthermore, in the presence of arginine, replacement of Cysteic (Acid) by a neutral amino acid exhibited a strong suppressive effect in these experiments.

***General Formula 1*** **Y-X-Z**

**Wherein:**
- **Y =**: **R*, H, K, Cys(acid), G or D;**
- **X =**: **G*, A, Cys(acid), R, G, D or E; and**
- **Z =**: **Cys*, G, Cysteine, R, D, N or E.**

**indicates component of the RGCys(acid) binding motif of tripeptide in GRGCys(acid)TP (ALG-1001), which was used as a Positive Control.**

Based on the initial data set forth herein, certain structure/activity relationships are suggested in relation to specific changes made to the R-G-Cysteic Acid binding motif. For example, when the amino acid R (i.e., the Y Component) of the R-G-Cysteic(Acid) binding motif is replaced by a basic amino acid or acidic amino acid, the peptide's anti-neovascularization effects diminish, whereas in the presence of arginine in the binding motif aspartic acid as Component Y appears to promote the peptide's anti-neovascularization effects.

When amino acid G (i.e., the X Component) of the R-G-Cysteic Acid binding motif is replaced by a basic or acidic amino acid, the peptide's anti-neovascularization effects decrease. However, in the presence of arginine (a strong hydrogen bonding), two carbon length-space for hydrophobic interaction (Alanine and Aspartic Acid) may not influence the peptide's anti-neovascularization effects.

When Cys(Acid) (i.e., the Z Component) of the R-G-Cysteic(Acid) binding motif is replaced by a neutral amino acid, the peptide's neovascularization inhibiting activity increases whereas replacement of the 2 component by acidic or basic amino acids causes the neovascularization inhibiting activity to decrease.

All indications are that the R-G-Cysteic(Acid) of the oligopeptide Glycinyl-Arginyl-Glycinyl-Cysteic(Acid)-Threonyl-Proline (ALG-1001 or Luminate^{®}, Allegro Ophthalmics, LLC) is important for suppression of neovascularization. Also, addition of three parts taurine to one part of the Glycinyl-Arginyl-Glycinyl-Cysteic(Acid)-Threonyl-Proline (ALG-1001) improves the neovascularization suppressing activity.

It is to be appreciated that, although the invention has been described hereabove with reference to certain examples or embodiments of the invention, various additions, deletions, alterations and modifications may be made to those described examples and embodiments without departing from the intended spirit and scope of the invention. For example, any elements, steps, members, components, compositions, reactants, parts or portions of one embodiment or example may be incorporated into or used with another embodiment or example, unless otherwise specified or unless doing so would render that embodiment or example unsuitable for its intended use. Also, where the steps of a method or process have been described or listed in a particular order, the order of such steps may be changed unless otherwise specified or unless doing so would render the method or process unsuitable for its intended purpose. Additionally, the elements, steps, members, components, compositions, reactants, parts or portions of any invention or example described herein may optionally exist or be utilized in the absence or substantial absence of any other element, step, member, component, composition, reactant, part or portion unless otherwise noted. All reasonable additions, deletions, modifications and alterations are to be considered equivalents of the described examples and embodiments and are to be included within the scope of the following claims.

### Annex B

The following numbered clauses provide further disclosure of embodiments and options, and also indicate potential scopes of protection within the contemplation of the applicant.
1. A composition of matter comprising a peptide which consists of or includes an amino acid sequence having the formula

   **Y-X-Z**

   wherein:
   - Y: = R, H, K, Cys(acid), G or D;
   - X: = G, A, Cys(acid), R, G, D or E; and
   - Z: = Cys(acid), G, C, R, D, N or E.
2. A composition according to clause 1 wherein the amino acid sequence is R - G - Cys (acid).
3. A composition according to clause 2 wherein the peptide comprises R - G - Cys (acid).
4. A composition according to clause 2 wherein the peptide comprises R - G - Cys (acid).
5. A composition according to clause 2 wherein the peptide comprises R - G - Cys(acid)-G-G-G-D-G.
6. A composition according to clause 2 wherein the peptide comprises Cyclo-{ R-G-Cys(acid)-F-N-Me-V}.
7. A composition according to clause 1 wherein the amino acid sequence is R - A - Cys (acid).
8. A composition according to clause 1 wherein the amino acid sequence is R - G - Cysteine.
9. A composition according to clause 1 wherein the amino acid sequence is R - Cys(acid) - G.
10. A composition according to clause 1 wherein the amino acid sequence is Cys(acid) - R - G.
11. A composition according to clause 1 wherein the amino acid sequence is Cys(acid) - G - R.
12. A composition according to clause 1 wherein the amino acid sequence is R - G - D.
13. A composition according to clause 12 wherein the peptide comprises Cyclo-{R-G-D-D-F-NMe-V}.
14. A composition according to clause 1 wherein the amino acid sequence is H - G -Cys(acid).
15. A composition according to clause 1 wherein the amino acid sequence is R - G - N.
16. A composition according to clause 1 wherein the amino acid sequence is D - G - R.
17. A composition according to clause 1 wherein the amino acid sequence is R - D - G.
18. A composition according to clause 1 wherein the amino acid sequence is R - A - E.
19. A composition according to clause 1 wherein the amino acid sequence is K-G-D.
20. A composition according to any of clauses 1 through 19 wherein the peptide comprises a salt.
21. A composition according to clause 20 wherein the salt is selected from trifluoroacetate, acetate and hydrochloride salt forms.
22. A composition according to any of clauses 1 through 19 further comprising taurine.
23. A composition comprising G-R-G-Cys(acid)-T-P or a salt thereof in combination with Taurine.
24. A method for inhibiting neovascularization or the development of pathological or aberrant blood vessels in a human or animal subject who is in need thereof, said method comprising the step of administering to the subject a therapeutically effective amount of a composition according to any of clauses 1-19.
25. A method according to clause 24 wherein the composition further comprises taurine.
26. A method according to either of clauses 24 or 25 wherein the method is carried out to inhibit neovascularization or the development of pathological or aberrant blood vessels in the eye.
27. A method according to clause 26 wherein the method is carried out to treat a disease or disorder selected from:: diabetic retinopathy, neovascular age-related macular degeneration, retinopathy of prematurity (ROP), sickle cell retinopathy, retinal vein occlusion, ischemia-induced retinopathy and inflammatory diseases of the eye.
28. A method according to clause 24 wherein the method is carried out to inhibit neovascularization development of pathological or aberrant blood vessels outside of the eye.
29. A method according to clause 28 wherein the method is carried out to inhibit the growth or metastasis of a vascularized tumor.

## Claims

1. A compound comprising or consisting of either:
a) an amino acid sequence having the formula:
**Gly-X-Thr-Pro**
wherein X is a three amino acid sequence selected from: Arg-Gly-Cys; Arg-Ala-Cys(Acid); Arg-Asp-Gly; Arg-Ala-Glu; Arg-Gly-Asn; Asp-Gly-Arg; Cys(acid)-Gly-Arg and Lys-Gly-Asp; or
b) the amino acid sequence Arg-Gly-Cys(acid)-Gly-Gly-Gly-Asp-Gly.

2. A compound according to claim 1 comprising the amino acid sequence Gly-Arg-Gly-Cys-Thr-Pro.

3. A compound according to claim 1 comprising the amino acid sequence Gly-Arg-Ala-Glu-Thr-Pro.

4. A compound according to claim 1 comprising the amino acid sequence Gly-Asp-Gly-Arg-Thr-Pro.

5. A compound according to claim 1 comprising the amino acid sequence Arg-Gly-Cys(acid)-Gly-Gly-Gly-Asp-Gly.

6. A compound according to claim 1 comprising the amino acid sequence Gly-Lys-Gly-Asp-Thr-Pro.

7. A compound according to claim 1 comprising the amino acid sequence Gly-Arg-Ala-Cys(acid)-Thr-Pro.

8. A compound according to claim 1 comprising the amino acid sequence Gly-Arg-Asp-Gly-Thr-Pro.

9. A compound according to claim 1 comprising the amino acid sequence Gly-Arg-Gly-Asn-Thr-Pro.

10. A compound according to claim 1 comprising the amino acid sequence Gly-Cys(acid)-Gly-Arg-Thr-Pro.

11. A salt form of compound according to any of claims 1 through 10.

12. A compound according to claim 11 wherein the salt form is selected from trifluoroacetate, acetate and hydrochloride salt forms.

13. A pharmaceutical preparation comprising a compound according to any of claims 1 through 12 in combination with at least one pharmaceutically acceptable carrier, diluent, solvent or excipient.

14. A pharmaceutical preparation according to claim 13 for use deterring retinal neovascularization in an eye of a mammalian subject.

15. A pharmaceutical preparation according to either of claims 13 or 14 suitable for intravitreal injection.
